# EUROPEAN PATENT APPLICATION

(11) **EP 1 375 459 A1**
(43) Date of publication of application: **02.01.2004**
(21) Application number: 02077576.3
(22) Date of filing: 28.06.2002
(51) Int. Cl.: C07C 7/144

(54) **Separation of unsaturated hydrocarbons from a fluid mixture**

(71) Applicant: Nederlandse Organisatie voor Toegepast-Natuurwetenschappelijk Onderzoek TNO, 2628 VK Delft (NL)
(72) Inventor: Weesling, Matthias, 7531 ER Enschede (NL); Nymeijer, Dorothea Catharina, 7558 MS Hengelo (NL); Visser, Tymen, 7521 JJ Enschede (NL); Van Soest-Vercammen, Esther Lucia Johanna, 3811 HH Amersfoort (NL)
(74) Representative: Prins, Adrianus Willem

(57) **Abstract**

Process for the separation of an unsaturated hydrocarbon from a fluid mixture with at least one other hydrocarbon wherein the unsaturated hydrocarbon is separated from the fluid mixture in a gas/liquid membrane contactor by passing the fluid mixture over a selective gas separation membrane, said selective membrane at least consisting of (a) a porous support material and (b) a non-porous active layer comprising at least one material, preferably present at the side of the membrane where the absorbent liquid is passed, wherein the active layer at least consists of a material with a high affinity for the complexing agent, such as a cation-exchange material comprising anionic functionalities or a material partitioning preferentially the complexing agent.

## Description

The present invention relates to a process for the separation of an unsaturated hydrocarbon from a fluid mixture. The invention further relates to a membrane, suitable for the use in hydrocarbon separations and to a device for carrying out such separations.

Unsaturated hydrocarbons such as ethene, propene, butene and styrene constitute a very important link in chemical production processes (e.g. polymerisation processes). They mostly occur in mixtures with other hydrocarbons, from which they often have to be separated. Usually cryogenic conditions are applied for the separation of ethene/ethane, propene/propane, butene/butane, styrene/ethylbenzene, etc. These separation processes are costly, however, both in terms of capital expenditure and operational costs. This is bound up with the fact that there is little distance between the boiling points of these hydrocarbons. In fact this is also the case with other chemical substances, for instance in the separation of nitrogen and oxygen. Since the thirties already it has been known that unsaturated hydrocarbons have the property of complexing reversibly with metals and metal ions, in particular with transition metal ions such as Ag⁺ and Cu⁺. This has given rise to some separation processes based on extraction. A well-known technique for application of reversibly complexing substances in a separation process is to immobilize them in the pores of a microporous membrane. Such a membrane is called an immobilized liquid membrane or facilitated transport membrane. Facilitated transport membranes for the separation of unsaturated and saturated hydrocarbons have received very much attention since the seventies. Later, other research groups have focused on this type of membrane. This separation method is based on the use of microporous membranes, with silver salts (mostly nitrate, sometimes tetrafluoroborate) applied in the pores in an aqueous solution, sometimes supplemented with a thickening agent to inhibit leakage. The separation is effected under atmospheric conditions at both sides of the membrane, as a pressure drop would entail loss of complexing liquid. A flow of sweep gas (often nitrogen) is passed at the back of the membrane in order to pick up the unsaturated hydrocarbon, which is preferably permeating. In the circumstances of industrial practice the process conditions are undesirable; thus, ethene/ethane mixtures are often under a pressure of about 2 MPa, while reducing this pressure to 0.1 MPa would entail high compression costs afterwards. Moreover, the ethene has to be separated again from the sweep gas. Further, the membranes appeared to have a short life as the permeating ethene is saturated with water vapour leading to a drying out of the membrane.

As an alternative to facilitated transport membranes the application of microporous membranes has in general been proposed, with the complexing or extracting liquid contained between two membranes. Also in this case, the process is only carried out under atmospheric conditions and with application of a sweep gas as otherwise loss of complexing liquid would occur.

In order to make this type of separation processes suitable for commercial and economic application it is required that they can be carried out at elevated pressure (0.2-5 MPa), the permeate has to be discharged, preferably without sweep gas, preferably at elevated pressure, the membrane has to be stable and should not lose its activity through loss of complexing agent and/or solvent, the process should generate sufficient flux and exhibit selectivity, and the product should be minimally contaminated with solvent.

US-A-5,057,641 and US-A-5,131,928 describe processes in which facilitated transport membranes are used and in which elevated pressures are applied, the permeate can be discharged without sweep gas and the membrane does not lose its activity. Due to the use of hydrophilic membranes and pores of a size of 10 to 200 Å, the capillary forces retaining the aqueous silver salt solution in the pores appear to be so great that at pressures of up to approx. 2.1 MPa the liquid is not expelled. Thus the process can handle liquid flows under pressure and the permeate flow can be discharged at reduced pressure and without sweep gas. Desiccation of the membrane is prevented by moistening the feed flow and by alternately or continuously passing at the permeate side the same complex solution as present in the pores. The flow permeating through the membrane is thus also present in said solution, from which it is separated in a flash drum at strongly reduced pressure.

Drawbacks of the above method included the slow transport, due to the sluggish diffusion of the resulting complex in the membrane (application of very narrow pores required in view of stability makes this diffusion additionally sluggish) and that very high compression costs are involved. As the permeating gas is discharged from the flash drum at reduced pressure and the pressure of this flow has to be restored, these separation processes are costly, while the gas discharged from the flash drum is saturated with water vapour, which entails an additional costly separation step. In addition, it tends to be problematic to discharge of the product at elevated pressure, to generate sufficient flux and selectivity and to avoid contamination with water.

In order to overcome these drawbacks, EP-A 0 634 204 proposes a process for the separation of an unsaturated hydrocarbon from a fluid mixture with other hydrocarbons wherein in a first stage the unsaturated hydrocarbon migrates through a membrane to a carrier fluid and in a second stage the unsaturated hydrocarbon is separated from the carrier fluid and removed, wherein in the first stage the fluid mixture is passed at superatmospheric pressure to one side of a first semi-selective gas separation membrane with a non-porous active layer, and a liquid complexing agent is passed along the other side of said first membrane, where said unsaturated hydrocarbon is bound through complexation in the interface of membrane and complexing agent, in that in the second stage said unsaturated hydrocarbon is dissociated from the complexing agent through temperature increase, in that the mixture of complexing agent and dissociated unsaturated hydrocarbon is separated and in that the complexing agent is recycled. The membrane is semi-selective, in that it selectively is impermeable to the liquid solvent (water) forming the carrier fluid (and comprising the complexing agent) but permeable to both the unsaturated hydrocarbon and the saturated hydrocarbon.

However, in the separation of unsaturated hydrocarbons from a fluid - in particular in olefin/paraffin separation - there are two important limiting cases for the olefin/paraffin selectivity in circulatory membrane absorbers used in gas/liquid membrane contactors. At low liquid flow rates the selectivity is determined by the selectivity of the transition metal solution, e.g. a silver nitrate solution, but the total productivity (which may also be referred to as flux) is low due to liquid side polarization. At high liquid flow rates, the productivity is high, but the selectivity is determined by the selectivity of the membrane. Thus one has to compromise between a high selectivity (by employing a low liquid flow rate) and a high productivity selectivity (by employing a high liquid flow rate).

It is an object of the present invention to provide an novel process for separating one or more unsaturated hydrocarbons from a fluid mixture, which process may serve as an alternative for known processes.

It has now been found that this object can be achieved by employing a membrane based upon a polymeric material, such as a membrane mentioned in EP-A 0 634 204, which membrane is provided with an active layer of a specific nature.

Accordingly, the present invention relates to a process for the separation of at least one unsaturated hydrocarbon from a fluid mixture with at least one other hydrocarbon wherein the unsaturated hydrocarbon is separated from the fluid mixture in a gas/liquid membrane contactor by passing the fluid mixture over a selective gas separation membrane, said selective membrane at least consisting of a porous support material, having interconnected pores and being permeable to the unsaturated hydrocarbon, and a non-porous active layer, preferably present at the side of the membrane where the fluid mixture is passed, wherein the top layer at least consists of a material with a high affinity for the complexing agent (preferably a transition metal ion), such as a polyelectrolyte having cation-exchange properties (provided by anionic functionalities) or a non-charged polymer showing extended partitioning of the complexing agent.

It has been found that with a process according to the invention, the membrane maintains to determine the selectivity of the separation process at a higher absorbant liquid velocity than with a similar membrane that does not comprise the non-porous active layer with a high affinity for the complexing agent. Thus higher selectivity can be obtained at a specific productivity or a higher productivity can be obtained at the same selectivity.

The term selective membrane is used herein to describe a membrane comprising an active layer that is substantially impermeable to the solvent of the absorbent liquid (such as water) and which furthermore has a higher permeability to the unsaturated hydrocarbon or hydrocarbons (bound to the complexing agent) than to the saturated hydrocarbon or hydrocarbons from which the unsaturated hydrocarbon is separated..

The term polyelectrolyte is defined herein as a polymer having ionisable groups, in particular groups that can be negatively charged, such that the polyelectrolyte is suitable as a cation-exchange material for the complexing agent. Examples of suitable polyelectrolytes are ionomers comprising a hydrophobic backbone provided with anionic functional groups.

A mixture of a non-charged polymer and an ionic complexing agent is also referred to herein as polymer electrolyte. Suitable polymers for forming the polymer matrix of the polymer electrolyte are polar polymers Examples of polymer matrices for polymer electrolytes are polar polymers with functionalities having moieties with a dipole moment, preferably a negative dipole moment..

Preferred polymers used as a matrix for polymer electrolytes include polyalkylene oxides, in particular polyethylene oxide (PEO) and -polypropylene oxide (PPO), polyalkylene imines, in particular polyethylene imine (PEI), and poly-4-vinylpyridine. Examples of suitable polyelectrolytes are homopolymers and copolymers of polyethylene polyalkyloxazoline, in particular polyethyloxazoline, polyphosphazine and polybenzimidazol. In particular, A polyalkylene oxide and a polyalkylene amine have been found to be capable of given rise to a relatively high selectivity at a given productivity.

The term porous is used herein to describe a material that contains interconnected pores, preferably ranging from 1 nanometer to 50 micrometer and that is permeable to the absorbent liquid if the latter wets the porous membrane or is forced through the pores by applying a pressure. It is stressed that in practice, the membrane will usually not be penetrated by the absorbant liquid, due to the presence of the active layer.

The term non-porous is used herein to describe a material that is essentially impermeable to the adsorbent liquid, yet still permeable to the unsaturated hydrocarbon, the complexing agent and the complex of unsaturated hydrocarbon with the complexing agent.

A preferred process according to the invention claim 1, comprises a first and a second stage:
(i) wherein in the first stage (the absorption stage):
   - the fluid mixture is passed at one side of a selective gas separation membrane, said selective membrane at least consisting of a porous support material, and a non-porous top layer
   - an absorbent liquid comprising a complexing agent having affinity for the unsaturated hydrocarbon, is passed along the other side of the selective membrane; and
   - wherein the porous side of the membrane faces the fluid mixture and the non-porous side the absorbent liquid comprising complexing agent
   - wherein the complexing agent preferentially partitions into the non-porous selective layer
   - wherein said unsaturated hydrocarbon is bound to the complexing agent at the interface of the selective membrane and the fluid mixture (also referred to as feed stream or feed mixture);
(ii) wherein in the second stage (the desorption stage),
   - the bound unsaturated hydrocarbon is dissociated from the complexing agent; and
   - the dissociated unsaturated hydrocarbon is separated from the absorbent liquid.

In the first stage, the fluid mixture can be passed at the membrane at subatmospheric, atmospheric or superatmospheric pressure. Preferably a superatmospheric pressure is applied.

For economic and environmental purposes, preferably at least part of the absorbent liquid or at least part of the complexing agent is recycled. Preferably the mixture of complexing agent and dissociated, unsaturated hydrocarbon is passed, *e,g.* at superatmospheric pressure, to one side of a second semiselective membrane which may or may not have a non-porous active layer (which membrane may be a semi-selective membrane as described EP-A 0 634 204, another type of a membrane used in the art or a selective membrane as described herein) wherein the unsaturated hydrocarbon migrates to the other side of the membrane and is discharged. An advantage of the present invention is that the process of absorption (the complexation reaction) is separate and independently adjustable from the process of desorption (the dissociation reaction); *e.g.* by using a temperature increase, a substantial dissociation can be obtained. The complexation reaction thus takes place behind the -selective membrane with a non-porous top layer in the interface of membrane and permeate flow.

The non-porous active layer preferably at least consists of a polymer film (optionally applied onto a porous carrier) in which the distances between the polymer chain segments usually vary from 2 and 20 Å 'Active' relates to the layer that determines the membrane activity. This active layer preferably is at the side of the absorbent liquid comprising the complexing agent, more preferably it is the top layer. Thus the active layer acts as a barrier prohibiting the wetting of the porous part of the membrane, which is undesired since it would result in increased diffusion resistance of the olefin through the total membrane.

Such membranes are usually very pressure resistant (in some cases to pressures higher than 10 MPa).

The fluid mixture may contain one or more saturated and unsaturated hydrocarbons. A process according to the invention can be used to separate paraffins from monoolefins, diolefins or acetylenes; diolefins from monoolefins; or acetylenes from paraffins, monoolefins or diolefins; as well as to separate a given aliphatically-unsaturated hydrocarbon from another of such materials in the same class where the members have differing complexing rates with the complexing agent. The feed need only contain a small amount of unsaturated hydrocarbon, as long as the amount is sufficient so that the unsaturated material to be separated selectively reacts with the metal complex to a significant extent, and thus at least one other component of the feed is less reactive or non-reactive with the complex-forming metal. The aliphatically-unsaturated materials of most interest with regard to separation by the method of the present invention have two to about twelve carbon atoms, preferably two to eight carbon atoms. The separation of aliphatically-unsaturated hydrocarbons from admixtures containing other hydrocarbons, such as the separation of ethene from ethane and methane, is of particular importance. Frequently such feed mixtures for the process contain about 1 to 50 weight percent ethene, about 0 to 50 weight percent ethane and about 0 to 50 weight percent methane. Propene is also an olefin in high demand, and its separation may be accomplished in accordance with the present invention. Another process that may be of special significance is the separation from ethene of minor amounts of acetylene. The process of the invention can also be used for the separation of aromatics from paraffins or cycloparaffins.

In the present invention those metals which can serve in the form of metal-containing cations to separate unsaturated hydrocarbons in the feed mixture through the formation of metal complexes of desired properties include, for instance, the transition metals of the Periodic Table of Elements having atomic numbers above 20. Included in these metals are those of the first transition series having atomic numbers from 21 to 29, such as chromium, copper, especially the cuprous ion, manganese and the iron group metals, e.g. nickel and iron. Others of the useful complex-forming metals are in the second and third transition series i.e. having atomic numbers from 39 to 47 or 57 to 79. Noble metals may be employed such as silver, gold and the platinum group, among which are platinum, palladium, rhodium, ruthenium and osmium. The useful base metals of the second and third transition series include, for example, molybdenum, tungsten, rhenium and the like. Various combinations of these complexing-forming metals may also be employed in this invention, either in the presence or absence of other non-metal or non-complexing metal cations. The concentration of the complexing agent is generally above 0.1 molar, preferably between 0.5 and 15 molar. Care should be taken that the concentration is always below the saturation point of the solution.

Very good results have been achieved with a absorbent liquid comprising a salt of silver or copper. Silver ions are particularly preferred as complexing agent.

The counter ion(s) for the complexing agent (if it is ionic) is not particularly critical in particular not when the active layer is base upon a cation exchange material. One may for example employ BF₄- or nitrate. A preferred counter ion in case a cation exchange material is employed, is nitrate. Nitrate salts are generally relatively cheap and tend to dissolve well in water.

In the case of polymer electrolytes, such as PEO-based polymers, preferred counter ions include BF₄-, SCN-, ClO4-, CFSO3-, Cl-, Br-, I-, and NO3- .In particular when the selective, non-porous active layer material at the feed side is essentially water free, AgBF4, AgSCN, AgClO₄ and AgCFSO3 is much preferred. It has been found that these silver salts also dissociateparticularly well in PEO in the absence of water.

The membranes generally at least consists of a porous support material and the active top layer. For the support material the following materials may be used: polysulfones, polyethersulfones, polyimides, polyacrylonitriles, PVC (polyvinyl chlorides), PE (polyethylenes), PP (polypropylenes, e.g. Accurel ®), PS (polystyrenes), polyamides. Preferentially the material is resistant to the solvent used to apply the non-porous selective active layer. The skilled person will know how to choose a suitable material for a specific solvent.

The active layer preferably comprises a polyelectrolyte and/or a polymer electrolyte. The polyelectrolyte and/or polymer electrolyte can be a homopolymer or a copolymer, in particular a block copolymer. Very suitable is a block copolymer. A preferred polymer in the active layer comprises a first type of blocks - with functionalities having affinity for the complexing agent- and a second type blocks, which are substantially inert to the absorption liquid and the complexing agent. A much preferred second type of blocks is a polymer block selected from the group consisting of polyalkylene terephtalate blocks. Very good results are achieved with polybutylene terephtalate (PBT) blocks. Polyalkylene terephtalate blocks, such as polybutylene terephtalate blocks have been found to improve the water-resistance of the active layer, which makes the membrane more durable.

A preferred active layer is based upon a cation-exchange material with anionic functionalities or a polymer electrolyte. Such materials have been found to give rise to a highly selective process, whilst allowing a satisfactorily high or even improved productivity. Preferred examples of polymer materials from which suitable cation exchange material can be made include polyetherketones, polyarylates, polysulphones, polyethersulfones, sulphonated perfluorocarbons and polyamides.

Good results have been achieved with a cation-exchange material based upon a polyether etherketon, e.g. PEEK (see formula 1, number 1-4 showing possible positions for an anionic functionality). However, any other material - in particular polystyrene, polyphenyleneoxide, polysulfone or polyethersulfone - may be functionalized by a cation-exchange group. It is clear to the expert that one may also choose any other polyelectrolyte such as a polyarcrylic acid, etc.

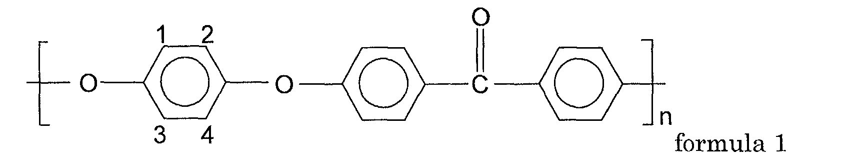

Polyetherketon based cation-exchange material has been found to have a relatively high permeability to gasses and a relatively low permeability to liquid, which prevents leakage of liquid into the pores of the support material. In addition the coating properties are good, in particular with respect to the avoidance of penetration into the pores of the support. Further, such material has been found highly stable under process conditions.

A particular advantage of polyetherketon based cation exchange material, e.g. sulfonated peek (SPEEK), is the relatively high permeability to complexing agent, such as Ag⁺, when the material is saturated with water.

Preferred examples of anionic functionalities include sulphonic acid groups, carboxylic acid groups, phosphoric acid groups and ionomers.

Particularly good results have been achieved with a cation-exchange material, e.g. based upon PEEK, comprising sulphonic acid groups.

The ion exchange capacity is preferably at least 0.5 mmol/g more preferably at least 1 mmol/g even more preferably at least 2 mmol/g.

Preferably, the ion exchange capacity is at most about 3 mmol/g, e.g. if a good resistance to dissolving in a (polar) absorbent liquid is important.

The active layer can consist of one or more polymers with functionalities having affinity for the complexing agent. Alternatively the active layer can be formed of a blend with one or more of such polymers and one or more other components, such as polymers that are insoluble in the absorbent liquid. Thus the resistance of the active layer against interacting with the absorbent liquid (in particular against dissolving therein) can be improved.

In practice, the membrane generally has an overall thickness of between 10 µ - 10 mm. The active layer preferably has a thickness of between 0,01-10 µ, more preferably between. 0.1 and 2 µm, even more preferably in the range of 0.1-1 µm Active layers with a thickness of less than 2 µm have been found to give rise to a relatively high productivity at a particular selectivity.

The form of the membrane may be any form that allows use in a process for separating unsaturated hydrocarbons from a fluid mixture. Very good results have been achieved with hollow fibre membranes. The surface area provided by these membranes is preferably in the range of 1000-8000 m²/m³ fluid mixture.

The temperature at which the absorption (the complexation) is to be performed is above the melt temperature of the absorbent liquid and generally between -10 and 50°C.

The desorption (dissociation) may take place at the same or a different temperature as the absorption temperature at which the desorption (the dissociation) is performed is preferably higher than the absorption temperature, more preferably at least 5°C higher. For practical reasons, the desorption temperature generally is between 10 and 150°C.

The pressure at which the process of the present invention can be performed is not critical and can be the same for absorption and desorption; a pressure reduction from absorption to desorption is also possible. For practical reasons, the pressures are generally between 0.05 and 5 MPa, preferably between 0.2 and 2 MPa. In the second stage use may be made of a sweep fluid, e.g. a sweep gas.

It is also possible to recover the unsaturated hydrocarbon(s) without a sweep fluid, thus avoiding the need to separate the hydrocarbon from the sweep fluid. Very good results have for example been achieved by using a pressure significantly different from the absorption pressure, e.g. a vacuum, to recover the unsaturated hydrocarbon.

To avoid potential reduction of the complexing agent during the process, the carrier fluid may contain or be supplemented with an oxidator, like nitric acid or hydrogen peroxide. Also other additives, e.g. to lower the water activity, can be used.

The invention also relates to a device with which the process described above can be carried out. This device comprises a first system of semiselective gas separation membranes comprising a non-porous active layer, means with which a fluid mixture is supplied at preferably a superatmospheric pressure to one side of the membranes, means with which a absorbent liquid comprising a complexing agent is supplied to the other side of the membranes, optionally with means with which to heat absorbent liquid with bound unsaturated hydrocarbon which has flowed along the other side of the membrane and means to separate the unsaturated hydrocarbon from absorbent liquid and to recycle the absorbent liquid/ complexing agent. Preferably the device further comprises a second system of gas separation membranes comprising a non-porous active layer, means with which at preferably a superatmospheric pressure the optionally heated absorbent liquid and the unsaturated hydrocarbon dissociated from it are passed along one side of the membranes of the second system, means with which to remove the unsaturated hydrocarbon that has migrated through said membranes of the second system.

The present invention further relates to a selective membrane suitable for separating an unsaturated hydrocarbon from a fluid mixture. A membrane according to the invention comprises a porous support material as defined above and a non-porous active layer, the active layer at least consisting of a cation-exchange material comprising anionic functionalities or a polymer electrolyte. More in particular the membrane at least consists of a porous support material as defined above and a non-porous layer of sulphonated, carboxylated or phosphonated poly(ether ether ketone). Membranes according to the invention have been found to be very suitable to separate unsaturated hydrocarbon from a fluid mixture. Very good results have been achieved with a membrane wherein the active layer is based upon PEEK, in particular sulphonated PEEK (SPEEK).

Preferably a membrane according to the invention is provided with silver ions or copper ions

Methods to prepare the membranes according to the invention or used in accordance with the invention are known in the art. For example the preparation of SPEEK is described in Bailly et al, Polymer 28 (1987), 1009-1016 but can be readily prepared by a person skilled in the art.

The active layer can be applied to the support material in any way known to the person skilled in the art. Very suitable is a dip-coating procedure. Herein the support material is dipped a number of times in a solution of the active material. If the support material is a hollow fibre, it is preferred to employ a coating method such that the inner side of the membrane should not be coated. This may be achieved by first preparing the hollow fibre membrane module with the support membrane only and performing the coating of the selective layer by a rinsing step subsequently. If SPEEK is the active material, a very suitable solvent is methanol. Alternatively, the active layer may be coextruded simultaneously with the porous support membrane during its production by a phase inversion process, *e.g.* as described in "Preparation of composite hollow fibre membranes: co-extrusion of hydrophilic coatings onto porous hydrophobic support structures, *Journal of Membrane Science, **In Press, Uncorrected Proof,** Available online 31 May 2002,* T. He, M. H. V. Mulder, H. Strathmann and M. Wessling"

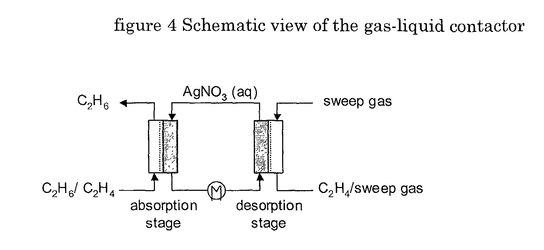

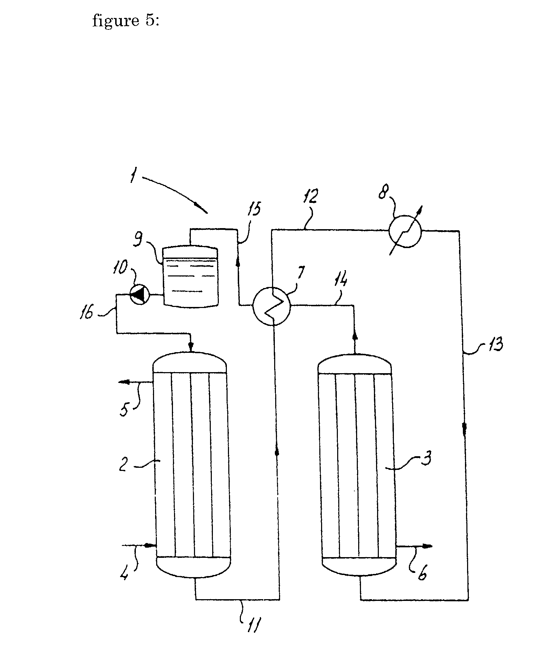

The invention will now be elucidated with reference to the schematic figures 4 and 5.

Figure 4 shows a schematic wherein a membrane contactor is used in an absorption/desorption process. A feed mixture (e.g. of alkenes and alkanes, such as ethylene and ethane) is brought into contact with a complexating agent (in an absorbent liquid) at the absorption stage, where the gas and the liquid phase are separated by the membrane (represented by the dotted line) . The absorbent liquid with the complexated olefin is transported to the desorption stage, where the olefin desorbs by changing the reaction conditions (changing the temperature or pressure difference). At the desorption stage, a second porous polymeric membrane is used to separate the liquid agent from the gas phase. The olefin is in this embodiment removed by a sweep gas.

Figure 5 shows an example of a device 1 with which a process according to the invention can be carried out. The device comprises a first separation unit 2 where the unsaturated hydrocarbon is absorbed into an absorbent liquid, and a second separation unit 3 where the unsaturated hydrocarbon is removed from the absorbent liquid. The separation units are optionally incorporated in a circuit 11-16 through which the absorbent liquid can be circulated by means of a pump 10.

Heating elements 7,8 are optionally integrated in the first part 11, 12, 13 extending from the first separation unit to the second one. At 8 fluid can be heated by means of a heating device, then it is passed through the second separation unit, whereupon the absorbent liquid is optionally passed to heat exchanger 7 via line 14 and flows on to buffer 9 via line 15. By means of heat exchanger 7 the heat of the absorbent liquid coming from the second separation unit can be used to preheat the carrier fluid coming from the first separation unit, whereby the fluid coming from the second separation unit can be cooled. In the buffer 9 the carrier fluid freed of the unsaturated hydrocarbon is collected, after which it may be circulated through the circuit 11-16 again via pump 10 and line 16.
The first separation unit further comprises feed lines 4 for the supply of a fluid mixture to be separated and means of discharge 5 for removal of a fluid mixture that has been depleted with the unsaturated hydrocarbon to be separated. The second separation unit 3 has means of discharge 6 for removal of the separated unsaturated hydrocarbon. The first and the second separation unit (2 resp. 3) are provided with membranes. In particular for unit 2 a system of hollow fibre membranes (high exchange areas) is preferred. The absorbent liquid, comprising a (preferably aqueous) solution with complexing agents such as silver and/or copper ions, is passed through the lumens of the hollow fibres. The fibres are equipped with the active layer at the outside of the fibres.

Via feed channels 4 a mixture of alkanes and alkenes is supplied to the first separation unit 2. The alkanes and alkenes can both move freely through the first membrane to the absorbent liquid. Both the alkanes
and alkenes are fractionally dissolved in the absorbent liquid, while the alkenes form a complex compound with complexing agent (e.g. the silver and/or copper ions). In this way the fluid mixture supplied via feed lines 4 is substantially freed of alkenes. Next the fluid mixture, in which the alkenes are bound in complexes, may be preheated in heat exchanger 7, supplied
to heating element 8 via line 12, where the absorbent liquid is heated further. As a consequence of the temperature increase the alkenes are dissociated from complexing agent, so that, when the absorbent liquid has been supplied to the second separation unit 3 via line 13, they can escape out of the absorbent by gas permeation and/or pervaporation via the second membrane, after which the product that is released, which substantially contains alkenes, is removed via the means of discharge 6.

The invention will be elucidated further by means of the following examples.

### Example 1: preparation of hollow fibre of sulphonated PEEK and ACCUREL®

### 1.1 Preparation of sulphonated PEEK

The sulphonation was done according to the method described by Jin et al [British Polymer Journal 17 (1985) 4-10]. The reactant, PEEK, was dried for 24 hours in a vacuum oven at a temperature of 30°C. Concentrated H₂SO₄ was heated to the desired temperature. The dried PEEK was dissolved in the concentrated H₂SO₄ to form 10 vol% solutions in a three-neck bottle. 36.6 g PEEK was needed for 200 ml H₂SO₄, which was the standard amount. The solution was stirred at a constant temperature for several hours. By cooling the solution for 15 minutes into an ice bath, the reaction was stopped. The cooled solution was precipitated with a dropping funnel into distilled water at a temperature of 0-5°C. The precipitated polymer was washed with fresh distilled water at a temperature of 0-5°C until the pH is about 5. At this point, the reaction was completely stopped. In all cooling steps, the temperature of the water may not exceed 10°C, since the polymer would (in some cases) dissolve above this temperature.

After the washing steps the polymer was dried in air overnight on glass plates. After one night the polymer was cut into small pieces with liquid nitrogen. In this way, the water evaporated faster. Finally, the pieces of polymer were dried in a vacuum oven at a temperature of 30°C for 48 hours.

The ion exchange capacity was 2.2-2.4 mmol/g (5 batches); the sulfonation degree was from 76-85 % (5 batches).Films of the SPEEK showed a completed swelling within 1 day.

The water uptake of SPEEK in 3.5 M silver nitrate was very low. To examine the influence of the concentration of silver nitrate on the water uptake, the swelling capacity of SPEEK when using a 2 M silver salt solution was measured. The water uptake corrected for exchanged silver ions was 20%, which is only slightly higher than with 3.5 M silver nitrate.

### 1.2 Preparing Composite Hollow Fibers of SPEEK and Accurel®

The SPEEK top layer was applied on the support by the dip coating technique [Mulder Basics of membrane technology, 2^{nd} ed Kluwer Academic Publishers, Dordrecht (1996)]. The support (Accurel®) was first sealed at one end, to prevent penetration of liquid into the bore. The other side was potted with glue (Epolam). A glass tube was filled with coating solution, which existed of 5 or 10 wt% SPEEK dissolved in methanol. The potted fibres were placed in the glass tube. After about ten seconds the fibres were withdrawn at constant speed out of this glass tube. A certain number of dip coating steps were carried out to ensure that the fibre is free of defects. After the first step, the potted side of the fibres was connected to a vacuum pump during coating. After every coating step the fibres were dried in air for at least one hour. After the whole dip coating procedure the fibres were dried in air over night.

The coated fibres were all defect free.

### Example 2

Membrane modules consisting of 10 composite hollow fibre membranes (prepared according to example 1 (inner diameter was 1.8 mm; outer diameter was 2.7 mm) with SPEEK top layers of 8-10 µm were prepared. The effective surface area of the modules was 101 cm². The performance of these modules was tested in a membrane contactor set-up with an absorption and a desorption stage.

A feed mixture of ethylene (80%) and ethane (20%) was used. Nitrogen was used as a sweep gas at the desorption side. The liquid flow rate of the 3.5 M silver nitrate solution was varied from 0 to 600 ml/min. Both absorption and desorption were carried out at room temperature. The permeate collected after desorption was analysed in a GC.

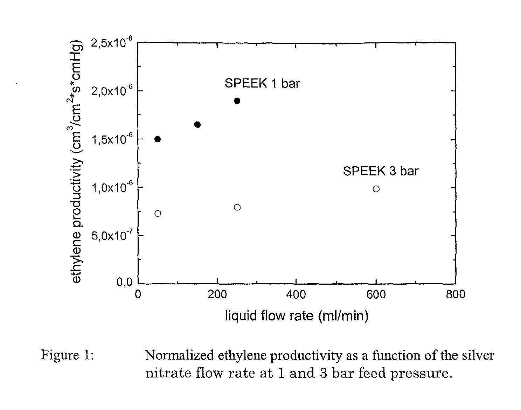

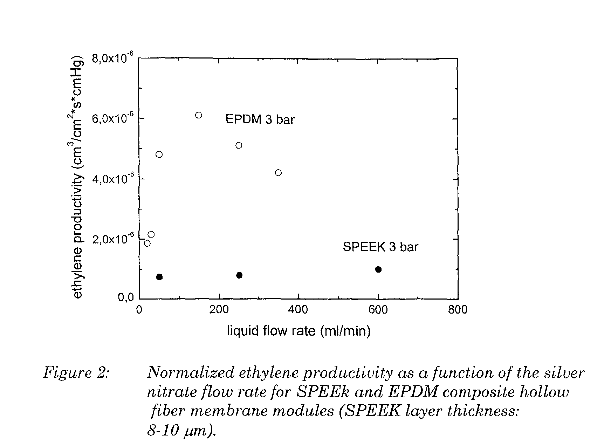

The influence of the feed pressure on the normalized ethylene productivity at different liquid flow rates is given in figure 1. Figure 1 shows that the normalized ethylene productivity increases with increasing silver nitrate flow rate. Furthermore, an increase in feed pressure results in a lower normalized ethylene productivity due to saturation limitations of the silver nitrate solution with ethylene. The ethylene productivity of the membrane modules containing membranes with a SPEEK top layer are compared to the same type of modules containing membranes with an ethylene propylene terpolymer (EPDM) top layer. Experiments are carried out at 3 bar and varying liquid flow rate. The results are given in figure 2.

A 100 times higher normalized ethylene productivity is observed for the EPDM coated membranes compared to the SPEEK coated membranes. A reduction of the SPEEK layer thickness resulted in a significant productivity increase. The lowered productivity can be understood from the nature of the polymer (being glass-like rather elastomeric).

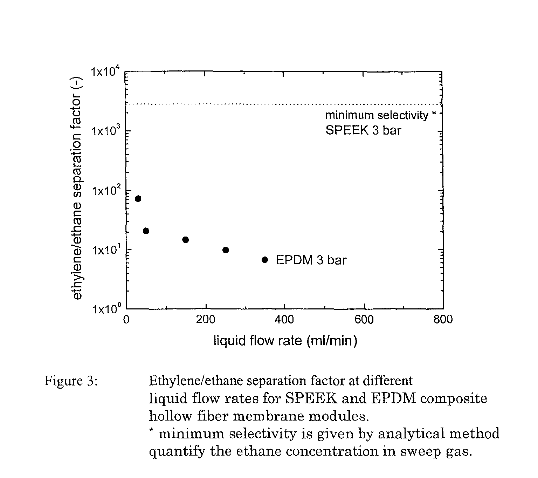

However, if the ethylene/ethane separation factor for the EPDM and SPEEK coated membranes are compared, it is clear that the separation factor of SPEEK coated membranes is 100-300 times the selectivity of the EPDM coated membranes (figure 3). Thus, it is clear that there is a net positive effect, if both selectivity and productivity are taken into account.

Due to the high selectivity, SPEEK and other materials capable of incorporating silver ions, seem to be very promising materials for membrane contactor applications. To increase the productivity materials with higher gas permeabilities could be used. Possible alternatives for SPEEK are for example other sulphonated polymers and polymers with -O- bond (*e.g.* PEO-PBT and poly(amide-6-b-ethylene oxide) (PEBAX))

## Claims

1. Process for the separation of an unsaturated hydrocarbon from a fluid mixture with at least one other hydrocarbon wherein the unsaturated hydrocarbon is separated from the fluid mixture in a gas/liquid membrane contactor by passing the fluid mixture over a selective gas separation membrane, said selective membrane at least consisting of (a) a porous support material and (b) a non-porous active layer comprising at least one material, preferably present at the side of the membrane where the absorbent liquid is passed, wherein the active layer at least consists of a material with a high affinity for the complexing agent, such as a cation-exchange material comprising anionic functionalities or a material partitioning preferentially the complexing agent.

2. Process according to claim 1, wherein
(i) in a first stage (the absorption stage):
- the fluid mixture is passed at one side of a selective gas separation membrane, said selective membrane at least consisting of a porous support material, and a non-porous top layer
- an absorbent liquid comprising a complexing agent having affinity for the unsaturated hydrocarbon, is passed along the other side of the selective membrane; and
- wherein said unsaturated hydrocarbon is bound to the complexing agent at the interface of membrane and fluid mixture;
(ii) and wherein in a second stage (the desorption stage),
- the bound unsaturated hydrocarbon is dissociated from the complexing agent; and
- the dissociated unsaturated hydrocarbon is separated from the absorbent liquid.

3. A process according to claim 1 or 2, wherein the non-porous active layer comprises a polyelectrolyte provided with anionic functionalities, wherein the polyelectrolyte is preferably based on a polyetherketone, a polyarylate, a polysulphone, polyethersulfone, a sulphonated perfluorocarbon or a polyamide.

4. A process according to claim 1 or 2, wherein the non-porous active layer is a polymer electrolyte

5. A process according to any of the preceding claims, wherein the cation-exchange material is a material comprising at least one type of anionic functionalities selected from the group consisting of sulphonic acid groups, carboxylic acid groups, phosphoric acid groups and ionomers.

6. A process according to any of the preceding claims, wherein the active layer comprises a polymer electrolyte selected from the group consisting of homopolymers and copolymers of polyalkylene glycols, preferably of polyethylene glycol, of polytalkylene imines, preferably polyethylene imine, of polyalkylene oxides, preferably polyethylene oxide, of polyalkyloxazoline, preferably polyethyloxazoline, of a polyphosphazine and of polybenzimidazol.

7. A process according to any of the preceding claims, wherein the support material is selected from the group consisting of : polysulfones, polyethersulfones, polyimides, polyacrylonitriles, PVC (polyvinyl chlorides), PE (polyethylenes), PP (polypropylenes), PS (polystyrenes), nylons.

8. A process according to any of the preceding claims, wherein in the second stage the dissociation is effected by changing the temperature and/or changing the pressure, preferably by increasing the temperature.

9. A process according to any of the preceding claims, wherein the complexing agent is recycled.

10. A process according to any of the preceding claims, wherein the complexing agent is selected from the group of silver ions and copper ions.

11. Process according to any of the preceding claims, wherein as a selective gas separation membrane is a hollow fibre membrane is used.

12. Process according to any of the preceding claims wherein in the first stage the fluid mixture is passed over the membrane at a superatmospheric pressure.

13. Device to carry out the process according to any one of the preceding claims, comprising:
a first system of selective gas separation membranes comprising a non-porous active layer, means with which a fluid mixture is supplied at preferably a super-atmospheric pressure to one side of the membranes, means with which a liquid complexing agent is supplied to the other side of the membranes, means with which to heat the complexing agent with bound unsaturated hydrocarbon which has flowed along the other side of the membrane, and means to separate the unsaturated hydrocarbon from the complexing agent and optionally means to recycle the complexing agent.

14. Device according to claim 13, further comprising a second system of semiselective gas separation membranes comprising a non-porous active layer, means with which at superatmospheric pressure the heated complexing agent and the component dissociated from it are passed along one side of the membranes of the second system and means with which to remove the component that has migrated through said membranes of the second system.
